# EUROPEAN PATENT SPECIFICATION

(11) **EP 4 329 851 B1**
(45) Date of publication and mention of the grant of the patent: **19.03.2025**
(21) Application number: 22720487.2
(22) Date of filing: 26.04.2022
(51) Int. Cl.: A61M 25/00, A61M 25/01

(54) **PACKAGED CATHETER ASSEMBLY**
VERPACKTE KATHETERANORDNUNG
ENSEMBLE DE CATHÉTER EMBALLÉ

(30) Priority: 27.04.2021 GB 202105987
(43) Date of publication of application: 06.03.2024
(73) Proprietor: ConvaTec Limited, Deeside, Flintshire CH5 2NU (GB)
(72) Inventor: JENCO, Vladimir, 071 01 Michalovce (SK); MECIAR, Braniclav, 071 01 Michalovce (SK); LACKO, Patrick, 071 01 Michalovce (SK); FENOVCIK, Peter, 071 01 Michalovce (SK)
(74) Representative: Wilson Gunn
(86) International application number: PCT/GB2022/051047
(87) International publication number: WO 2022/229620

(56) References cited:
- EP-A1- 3 590 573
- US-A1- 2015 352 321
- US-A1- 2017 007 802

## Description

### Technical Field of the Invention

The present invention relates to packaged catheter assemblies. In particular to packaged urinary catheter assemblies, and most particularly to packaged intermittent male urinary catheter assemblies.

### Background to the Invention

Many people suffer from urinary problems that can make it difficult to pass urine. Intermittent catheter assemblies can provide a convenient and portable solution to this problem as a user is able to self-catheterise to relieve themselves as required. This reduces the effect of their urinary condition on their life and allows thm to enjoy a relatively normal lifestyle.

Intermittent catheters are typically single-use devices that are carried by the user then unpackaged and used as required. When using an intermittent catheter in a public place, it is also advantageous to be able to re-package the catheter until a convenient time to dispose of the catheter. However, this can be difficult, especially for Male intermittent catheters which are generally several centimetres longer than female catheters, typically at least 20cm long, with ISO 20696:2018 setting a minimal effective length of 275mm. This can make them difficult to re-package in a discreet manner.

To reduce the risk of damage to the urinary tract and improve comfort while using the catheter, it is also important they are lubricated before use. However, once lubricated and used a catheter can be slippery and difficult to handle. This can make re-packaging of the catheter after use difficult and unhygienic, as the used catheter may inadvertently contact the user's hands, clothing or personal items.

Existing intermittent catheters such as the Coloplast SpeediCath Flex (RTM) and the Hollister VaPro (RTM) catheters disclose continuous handling sleeves that cover the entire length of the catheter. While these provide some assistance to handling, the sleeve must be bunched up against one end of the catheter during use which can be difficult and unhygienic as dirt may get onto the catheter prior to use during bunching of the sleeve. Examples of packaged catheters can be found in US- A-2015/352321, US-A-2017/007802 or EP-A-3590573.

It is an object of embodiments of the present invention to at least partially overcome or alleviate the above problems.

### Summary of the Invention

The invention is defined in the claims.

Advantageously, the present invention provides for a handling sleeve that provides at least two handling sleeve elements which allow for more convenient and simpler handling of the catheter before, during, and after use. This can reduce the need to adjust the sleeve prior to use of the catheter, reducing the chance of dirt contacting the catheter and associated infection. Furthermore, the handling sleeve elements provide greater flexibility in terms of handling the catheter than a single long sleeve, this makes the catheter easier to re-package into the pouch after use.

According to a first aspect, the catheter comprises one handling sleeve configured to provide at least two handling sleeve elements. Accordingly, in one example the catheter comprises one handling sleeve configured to provide two handling sleeve elements. Alternatively, the catheter may comprise two or more handling sleeves. Where there are two or more handling sleeves, each handling sleeve may be configured to provide at least one handling sleeve element. Thus, the handling sleeves and handling sleeve elements can be flexibly configured to allow for easier handling of the catheter as they can be optimised for the specific packaging requirements of a given catheter/pouch. They can also therefore be configured to suit users with different levels of mobility and dexterity.

The two handling sleeve elements are preferably separate. At least one handling sleeve is configured to separate into at least two handling sleeve elements. Thus, two or more handling sleeve elements may be retained in a convenient position prior to use as they form part of the same handling sleeve, this makes the catheter easier to use and the handling sleeve elements easier to access. The provision of a separable handling sleeve can also make manufacture of the packaged assembly simpler whilst still providing the benefits of at least two handling sleeve elements.

At least one handling sleeve may comprise one or more points of weakness to facilitate separation of the sleeve into at least two handling sleeve elements. The or each point of weakness may be one or more perforations. The one or more perforations may be provided about a circumference of the sleeve. The perforations may extend circumferentially around the circumference of the sleeve. In one example, the catheter may comprise one handling sleeve and the handling sleeve may be separable at the perforations to provide two handling sleeve elements. Thus, the handling sleeve may be easily separated by tearing the handling sleeve along the perforations. This facilitates easy separation without additional tools and the separation process does not create unnecessary loose parts that can be difficult and inconvenient to repackage back into the pouch after use.

The perforations may comprise a row of holes extending around the circumference of the handling sleeve. The holes may be separated by a distance that is no more than the size of each hole in a direction parallel to the circumference of the respective handling sleeve. The holes may each have a width and a length perpendicular to a width and a length of the handling sleeve (as defined below) respectively. The holes may be elongate. The aspect ratio (length:width) of each hole may be at least 1.5:1, 2:1, 3:1, 4:1. The holes may be separated by a distance that is no more than 100%, 90%, 80%, 70%, 60%, 50%, 40%, 30%, 20% or 10% the length of each hole. The separation of adjacent holes may depend on the length of the holes, alternatively the separation of adjacent holes may be constant irrespective of the length of the holes. The holes may be separated by a distance that is less than 4 mm, 2 mm or 1 mm. The holes may be separated by a distance that is at least 0.4 mm, 1 mm, or 2 mm. The length of each hole may be at least 0.5 mm, 1 mm, or 2 mm. The length of each hole may be less than 6 mm, 2 mm, 1 mm, or 0.5 mm. Preferably, the length is 2 mm, and the holes are separated by a distance of 1 mm. As such, the perforations provide for a point of weakness in the handling sleeve that is sufficient to allow easy separation of the handling sleeve into handling sleeve elements.

Each hole forming the perforations may be a slit. Where each hole is a slit, it may be formed by a one dimensional cut in the handling sleeve. Where each hole is a slit it may have an aspect ratio (length:width) that is effectively 1:0. Each cut may be made over a distance corresponding to the desired length of each hole. Alternatively, the slitted perforations may be formed in any other suitable way. In some embodiments, each slit may be formed by attaching two or more handling sleeve elements end-to-end, and sealing adjacent handling sleeve elements at locations that do not correspond to the locations of the slits. Advantageously, where the holes are slits, the catheter is less likely to be exposed through the handling sleeve than if the hole was not a slit. This helps prevent inadvertent contamination of the catheter while handling.

The point of weakness may be provided by a tear-off strip. The point of weakness may be provided by a peel-off adhesive. The point of weakness may be provided by sealed hook-and-hook or hook-and-loop elements. Thus, the point of weakness may be configured in a variety of different ways.

At least one handling sleeve may form a sheath around at least part of the catheter. At least one handling sleeve may be tubular, for example cylindrical. At least one handling sleeve may be configured to be separable at a point along its axis. At least one handling sleeve element may form a sheath around only part of the catheter. At least two handling sleeve elements may each form a sheath around only part of the catheter. At least one handling sleeve element may be tubular, for example cylindrical. At least two handling sleeve elements may each be tubular, for example cylindrical. Thus, the handling sleeve provides a sheath around the catheter and may be separated into handling sleeve elements that also provide a sheath around parts of the catheter facilitating easy handling of the catheter without the user having to touch the catheter directly.

At least one handling sleeve may be formed of one or more strips of material joined together along their edges. The join may be provided by any suitable means such as a weld; mechanical seal; heat seal; pressure seal; adhesive; solvent bond; ultraviolet bond; ultrasonic weld; laser weld; impulse weld; or friction weld. The strips of material may be elongate. Where the strips of material are elongate, they may be joined along their elongate edges. The strips of material may be trapeziums. Thus, the handling sleeve can be easily manufactured in a production line by welding the elongate sides of each strip of material together. This can remove the need for the catheter to be fed through the sleeve during manufacture which is a complex process.

A point of weakness in a handling sleeve may be formed after formation of the handling sleeve. In such embodiments, this may be convenient where the handling sleeve or each handling sleeve element is formed from a single/the same type of material. In embodiments where the point of weakness is perforations, the perforations may be cut into the handling sleeve. Alternatively, a point of weakness may be formed during formation of the handling sleeve. In some embodiments, the handling sleeve may be formed from two or more handling sleeve elements joined end-to-end. This may be convenient where the handling sleeve or each handling sleeve element is formed from different strips of material. In one example, the point of weakness may form the end-to-end join between adjacent handling sleeve elements. For example, the ends of the respective handling sleeve elements may be intermittently welded together to form perforations at the join. In other examples, any suitable joining means may be used such as mechanical seal; heat seal; pressure seal; adhesive; solvent bond; ultraviolet bond; ultrasonic weld; laser weld; impulse weld; or friction weld. Thus, the points of weakness can be conveniently formed in the or each handling sleeve.

Each handling sleeve may have a length measured parallel to the axis of the catheter, and a width measured perpendicular to the axis of the catheter. The width of each handling sleeve may be constant along at least part of its length. The width of the handling sleeve may vary along at least part of its length. The length of the handling sleeve may be less than: 275 mm; 250 mm; 200 mm; 150 mm; 125 mm; or 100 mm. The length of a handling sleeve may be at least: 50 mm; 100 mm; 125 mm; 150 mm; 200 mm; or 250 mm. Preferably, the length of a handling sleeve may be between 120-100 mm, more preferably 110 mm. The width of a handling sleeve may be less than: 60 mm; 40 mm; 30 mm; 25 mm; 20 mm; or 15 mm. The width of a handling sleeve may be at least: 10 mm; 15 mm; 20 mm; 25 mm; 30 mm; or 40 mm. Preferably, the width of a handling sleeve is between 15-25 mm. Thus, each handling sleeve, and each corresponding handling sleeve element, can be manufactured to ensure it has the correct width to ensure it is both easy to use and also closely fits the catheter.

Where the width of a handling sleeve is constant along at least part of its length, it may be formed from two rectangular strips of material. In one example, the rectangular strips of material are 110 mm in length and 19 mm wide. At least part of a handling sleeve may be a conical. The width of a conical part may increase or decrease along the length of the conical part of the handling sleeve. A conical part of a handling sleeve may be formed from strips of material that are isosceles trapeziums. Where the strips of material are isosceles trapeziums they may be joined together along their legs. In one example, the strips may have a length (i.e. height of an isosceles trapezium) of 110 mm, and a width that varies from 23 mm at one end to 17 mm at the other. Thus, the handling sleeves and handling sleeve elements can be conveniently manufactured with strips of material into a variety of different sizes to ensure good fit to the catheter and easy use. Where part of a handling sleeve is conical this has the added benefit that it, and any corresponding handling sleeve elements, are less likely to be inadvertently removed from the catheter due to the narrower width at one end. Furthermore, the wider end of the conical part can more easily fit over the ends of the catheter to allow for easy manipulation of them without the user having to touch them directly and without the handling sleeve coming off the catheter.

Each handling sleeve element may form part of a corresponding handling sleeve. Each handling sleeve element may be at least 20%, 30%, 40%, 50%, 60% or 70% the length of its corresponding handling sleeve. The length of a handling sleeve element may be at least 35 mm, 45 mm, 55 mm, 65 mm, or 75 mm, and preferably 55 mm. Where a handling sleeve comprises two or more handling sleeve elements, each handling sleeve element is preferably the same length. Alternatively, where a handling sleeve comprises two or more handling sleeve elements, one or more of the handling sleeve elements may be different lengths. Where a handling sleeve comprises two handling sleeve elements, the ratio of the length of the elements may be between 50:50 and 70:30, or more preferably between 50:50 and 60:40. Thus, the handling sleeve elements are sized to ensure the user has adequate space to hold each handling sleeve element before, during, and after use of the catheter.

The catheter may be a urinary catheter. The catheter may be a male urinary catheter. The catheter may be an intermittent catheter. In one embodiment, the catheter is an intermittent male urinary catheter. Thus, the features of the present invention allow intermittent male urinary catheters to be provided in a compact packaging that also facilitates convenient and easy withdrawal, use and re-packaging of the catheter.

The catheter may comprise a proximal end for insertion into the body and a distal end. The catheter may comprise a funnel at its distal end. The funnel may facilitate easier handling of the catheter. Where the catheter is a urinary catheter, the funnel may allow the user to better direct urine as it leaves the catheter during use. At least one handling sleeve and/or handling sleeve element may be configured to overly the funnel. At least one handling sleeve and/or handling sleeve element may be arranged at the distal end, for example around or next to the funnel prior to withdrawal of the catheter from the pouch. The distal end and/or funnel may be withdrawn from the pouch before the proximal end. Thus, the handling sleeve(s)/handling sleeve elements can be arranged such that they can be used to withdraw the catheter from the pouch, this helps ensure the catheter remains clean and sterile prior to use and reduces the risk of infection. Furthermore, as the handling sleeve elements can overly the funnel, they can reduce the likelihood of touching the end of the funnel during re-packaging the catheter after use. This makes using the re-packaging the catheter more hygienic for the user.

The handling sleeve(s) and/or one or more handling sleeve elements may be movable along the length of the catheter. Each handling sleeve element may be independently movable along the length of the catheter. One or more handling sleeves and/or handling sleeve elements may not be joined to the catheter. Preferably the or each handling sleeve, and each handling sleeve element may not be joined to the catheter. Thus, the catheter can be more conveniently handled by moving the handling sleeve elements along the catheter to suit the circumstances of the use and requirements of the user. Alternatively, one handling sleeve element may be moveable along the length of the catheter and one handling sleeve element may be joined to the catheter. In particular one handling sleeve element closest to the distal end of the catheter may be joined to the catheter and one handling sleeve element closest to the proximal end of the catheter may be moveable along the length of the catheter.

One or more of the handling sleeve elements may be configured to enable arrangement of the catheter within the pouch after use. The handling sleeve elements may comprise a proximal handling sleeve element and/or a distal handling sleeve element. The proximal handling sleeve element may be arrangeable at the proximal end of the catheter after use. The distal handling sleeve element may be arrangeable at the distal end of the catheter. The handling sleeve elements may comprise one or more handling sleeve elements arrangeable at a midpoint of the catheter, or closer to a midpoint than the ends of the catheter. The proximal handling sleeve element and/or the distal handling sleeve element may be arrangeable at a midpoint of the catheter, or closer to a midpoint than the ends of the catheter. In a preferred embodiment the handling sleeve elements comprise a distal handling sleeve element and a proximal handling sleeve element. Thus, the handling sleeve elements can be configured to be arrangeable in convenient locations along the catheter.

At least one of the two or more handling sleeve elements may be configured to receive and/or retain at least two sections of the catheter simultaneously. The two or more handling sleeve elements may be configured to retain the catheter in a looped or coiled configuration. Where a handling sleeve element receives at least a first and second section of the catheter, the catheter may be looped between the first and second sections. One or more handling sleeve elements may be configured to receive and/or retain an end of the catheter. Preferably, one or more handling sleeve elements may be configured to receive and/or retain a proximal end of the catheter. The distal handling sleeve element may be configured to receive and retain the proximal end of the catheter. Thus, the size/shape of the catheter can be restricted by the handling sleeves and ensuring the catheter can be easily repackaged back into the pouch. The use of two or more handling sleeve elements further allows for a more restricted shape of the catheter than would be possible with a single handling sleeve element.

At least part of the catheter may be configured to be wetted during withdrawal of the catheter from the pouch. At least part of any one or more of the at least one handling sleeves may be configured not to be wetted during withdrawal of the catheter from the pouch. At least part of each handling sleeve element may be configured not to be wetted during withdrawal of the catheter from the pouch. Less than 50%, 40%, 30%, 20%, or 10% of the length of a handling sleeve may be configured to be wetted during withdrawal of the catheter from the pouch. At least one handling sleeve element may be configured not to be wetted during withdrawal of the catheter from the pouch. Thus, the catheter may be adequately lubricated for use without significantly affecting the handling sleeve(s) or handling sleeve elements.

The handling sleeve(s) and/or handling sleeve elements may be formed of any suitable film materials. The handling sleeve(s) and/or handling sleeve elements may be impermeable to fluids. The handling sleeve(s) and/or handling sleeve elements may be made of a plastics material. The handling sleeve(s) and/or handling sleeve elements may be made from any one or more of: polypropylene (PP), low density polyethylene (LDPE), high density polyethylene (HDPE) or biaxially orientated polypropylene (BOPP), for example. The catheter and/or pouch may be made from the same or different materials as the handling sleeve(s) and/or handling sleeve elements. In a preferred embodiments, the catheter is a hydrophilic thermoplastic elastomer (TPE), and the handling sleeve(s) and/or handling sleeve elements are LDPE. Where the catheter comprises a funnel, the funnel may be polyvinyl chloride (PVC). Thus, the handling sleeve(s) and/or handling sleeve elements, and well as the catheter and funnel can be made from suitable materials.

The pouch may comprise two walls and a peripheral seal between the two walls. The catheter may be arranged within the pouch in a curved configuration. A join may be provided between the two walls of the pouch. The join and an internal base of the peripheral seal may define a channel to be filled with fluid prior to withdrawal of the catheter from the pouch. The pouch and catheter may be arranged such that during withdrawal of the catheter from the pouch, the distal end of the catheter is withdrawn from the pouch before the proximal end and the proximal end of the catheter passes through the channel. One or more handling sleeves may be arranged not to pass through the channel during withdrawal of the catheter from the pouch. Thus, the catheter may be removed and simultaneously lubricated without the one or more handling sleeves passing through the channel and becoming lubricated. Furthermore, any risk of the handling sleeve becoming damaged or inadvertently separating into one or more handling sleeve elements during withdrawal of the catheter is reduced as the handling sleeve does not pass through the channel.

The one or more handling sleeves may be arranged around a section of the catheter that is not intended for insertion into the body. The one or more handling sleeves may not be arranged around sections of the catheter intended for insertion into the body. Sections of the catheter not intended for insertion into the body may not be arranged to pass through the channel during withdrawal of the catheter from the pouch. Sections of the catheter intended for insertion into the body may be arranged to pass through the channel during withdrawal of the catheter from the pouch. Thus, the one or more handling sleeves do not inhibit the lubrication of the catheter whilst it passes through the channel during withdrawal of the pouch.

The packaged catheter assembly may comprise a fluid reservoir. The fluid reservoir may be configured to release fluid into the pouch to form a pool of fluid in the channel. The volume of the fluid reservoir is preferably sufficient to completely fill the channel, i.e. with the pouch held with its base lowermost, the fluid may extend to at least the level of the bottom of the join, so that the fill level of the channel is 100% or more, for example at least 150%, at least 200% or even at least 300%. Alternatively, the fill level of the channel may be: at least 70%, at least 80%, at least 90%; or at least 95% the volume of the channel. At least one handling sleeve element may be arranged above the fill level of the channel prior to withdrawal of the catheter from the pouch. Thus, the present invention provides for an assembly with a reservoir that is able to deliver fluid into the channel. The size of the reservoir is selected to ensure sufficient fluid is delivered and the catheter can be adequately lubricated on removal, in addition, at least one handling sleeve element remains above the fill level and remains in a dry state enabling more convenient handling of the catheter.

The pouch may be sealed. The fluid reservoir may be arranged within the (sealed) pouch. The fluid reservoir may be provided as a separate device, or incorporated into the pouch material. The fluid reservoir may comprise a deformable, frangible or burstable sachet. Thus the fluid reservoir is conveniently located within the pouch where it may directly release fluid into the channel formed by the join and the base of the peripheral seal. A deformable, frangible or burstable reservoir enhances the convenience for a user as fluid can be easily released without additional tools and before the pouch is unsealed, thus reducing the chances of accidental wetting of the user. A sachet in particular can be easily handled on an assembly line and introduced more simply than attempting to handle fluid, so as to it into the package during manufacture.

The catheter may be arranged between the join and the internal base of the peripheral seal. The join may be separate from the base of the peripheral seal. The channel may be an open-ended channel. Thus, the catheter is held within the pouch in the open-ended channel formed by the join and the internal base of the peripheral seal. As such, when withdrawn, the catheter is drawn through the channel and is adequately lubricated.

The join and the peripheral seal may be independent. The join may be provided in a spot. The join is preferably provided by a weld, e.g. a spot weld. Such a weld may be provided after the catheter (and optionally the sachet) have been arranged between the walls, and can be achieved from outside of the pouch. Alternatively, the join may be provided by any of a: mechanical seal; heat seal; pressure seal; adhesive; solvent bond; ultraviolet bond; ultrasonic weld; laser weld; impulse weld; or friction weld. Similarly, the peripheral seal be provided by any one or more of a weld; mechanical seal; heat seal; pressure seal; adhesive; solvent bond; ultraviolet bond; ultrasonic weld; laser weld; impulse weld; or friction weld. At least part of the peripheral seal may be formed by a fold. In such embodiments, a single sheet may be folded, for example at the base or one of two sides, to form two opposing walls of the pouch. The join can be provided in a single location allowing the peripheral seal and join to be formed separately. Alternatively, the weld forming the join may be provided at the same time as the peripheral seal. In some embodiments, the join and peripheral seal are a single continuous seal. The manufacturing process of the join can thus be very convenient and avoid major modification of a typical packaged catheter assembly production line.

The catheter may comprise a surface which is activated by the fluid in the channel. For example, the catheter may comprise a hydrophilic surface, formed for example by a hydrophilic coating. Where a hydrophilic surface is provided, the fluid may be polar or water-based. The catheter may comprise a hydrophobic surface, which is activated by the fluid in the channel. For example the catheter may comprise a hydrophobic coating. Where a hydrophobic coating is used, the fluid may be nonpolar. Thus the catheter is easily lubricated by fluid in the channel and is easy and comfortable to use.

The handling sleeve may comprise a surface which is not activated by the fluid. A surface of the handling sleeve may be hydrophobic. An inner surface of the handling sleeve may be hydrophobic. The handling sleeve may be embossed with a pattern. An inner surface of the handling sleeve may be embossed with a pattern. The pattern may be a linen pattern. Preferably an inner surface of the handling sleeve adjacent to the catheter in use is hydrophobic and embossed with a pattern. Advantageously, this reduces friction between the handling sleeve and the catheter in use. In some embodiments, the surface properties of parts of the handling sleeve, or individual handling sleeve elements may vary depending on their functionality. For example, where a part of the handling sleeve, or a handling sleeve element, is intended not to move along the length of the catheter, its surface properties may be selected to increase friction between the handling sleeve and the catheter. Thus, the surface properties of the handling sleeve can be selected to ensure the handling sleeve and catheter are easy to use.

The pouch may comprise an interaction region defining a first point of contact for a user of the packaged catheter assembly. The interaction region may facilitate user access to the contents of the pouch. Actuation of the interaction region may create an opening in or near an upper edge of the peripheral seal. The catheter may be withdrawn through the opening. Thus, access to the pouch is provided at or near an upper edge of the pouch and as such, the fluid in the channel (at the bottom of the pouch) is retained in the pouch while the catheter is withdrawn reducing the risk of spilling fluid outside the pouch.

The interaction region may comprise a breakable region of the peripheral seal. The method may comprise forming a breakable region in the peripheral seal. The breakable region may comprise a tear-away region which may comprise a tear line defining a line along which the pouch may be torn to at least partially separate the tear-away region from the remainder of the pouch, thereby creating an opening in the peripheral seal. The tear-away region may comprise a tear start. The tear start may be provided at a first end of the tear away region. The tear start may comprise a notch, or area of weakness, in one or both of the walls of the pouch. The tear start may define the first part of the pouch to be torn. The tear-away region may comprise a tear stop. The tear stop may be provided at a second end of the tear-away region. The tear stop may act to prevent or reduce the likelihood of the tear-away region being completely separated from the remainder of the packaging. The tear-away region may be substantially triangular in shape having a sloped upper edge. Thus the breakable region provides a convenient and easy means to open the pouch with minimal effort and no additional equipment/tools.

The interaction region may comprise a sealing arrangement. The sealing arrangement may comprise one or more sealing members operable to provide a user-sealable seal between the two walls of the pouch, so as to close the pouch. In one embodiment, first and second sealing members are provided on an interior surface of the two walls of the pouch. The sealing arrangement may provide a watertight and/or fluid impermeable seal when closed. Thus the pouch can be resealed to ensure the fluid in the channel is not subsequently released from the pouch after use. This also allows the catheter to be repackaged back into the pouch after use and retained within it.

The sealing arrangement may come pre-sealed. In such embodiments, user access to the contents of the pouch may be provided by opening the sealing arrangement. The sealing arrangement may be resealable. The sealing arrangement may be any of: a zip-lock seal; hook and loop seal; hook and hook seal; or adhesive layer. Therefore, the sealing arrangement can be used to ensure the contents of the pouch remain inside until the sealing arrangement is opened, and the pouch can still be resealed after use. This can help if the breakable region requires force to be applied to the pouch to be opened, as the pouch may move or jerk in position while being opened causing fluid or the catheter to exit the pouch.

The interaction region may comprise a breakable region and a sealing arrangement. In certain embodiments, the interaction region comprises a tear-away region corresponding to the upper edge of the peripheral seal, and a resealable arrangement below the tear-away region.

The peripheral seal may have an upper edge. The peripheral seal may be substantially rectangular in shape with two lateral edges between the base and upper edge. The peripheral seal may have a height from the base to the upper edge of at least 10 cm, 15 cm, or 20 cm; and/or a height of no more than 10 cm, 15 cm, 20 cm or 25 cm, such as between 15 cm and 20 cm. The peripheral seal may have a width between the lateral edges of at least 5 cm, 10 cm; or 15 cm; and/or a width of no more than 10 cm or 15 cm, or 20 cm such as between 5 cm and 15 cm. The catheter may have a length of at least: 20 cm; 25 cm; 30 cm; 35 cm; 40 cm; 45 cm; or 50 cm, for example between 35 and 45cm. In one embodiment, the peripheral seal has a height 10-25 cm and a width of 6-11 cm, and the catheter has a length of at least 30 cm. Thus, the peripheral seal defines a "pocket-sized" pouch that can store a catheter within in a curved configuration to reduce the apparent size of the catheter and enable convenient and discreet storage.

The at least one of the one or more handling sleeves may be arranged at or next to the distal end of the catheter. The distal end of the catheter may be arranged closer to an upper edge of the peripheral seal than the base of the peripheral seal. The distal end of the catheter may be arranged in the upper half of the pouch, or the upper quarter of the pouch. The distal end of the catheter may be arranged above the join. The distal end of the catheter may be arranged above the fill level of the fluid. Thus, the distal end of the catheter is easy to access and presents a handling sleeve to the user so that the catheter may be withdrawn conveniently using the handling sleeve. The handling sleeve is also positioned so as not to be pulled through the fluid, so will not be wetted and will remain easy to handle.

The distal end of the catheter may be provided with at least one handling sleeve, which may be arranged closer to an upper edge of the peripheral seal than the base of the peripheral seal. At least part of a handling sleeve may be arranged in the upper half of the pouch or the upper quarter of the pouch. At least part of a handling sleeve may be arranged above the join. At least part of a handling sleeve may be arranged above the fill level of the fluid. Thus, the handling sleeve is easy to access so that it may be withdrawn before the proximal end of the catheter as required by the invention. It is also positioned so as not to be pulled through the fluid, so at least part of it will not be wetted and will remain easy to handle, providing a dry surface that can be gripped and used to manoeuvre the catheter.

The join may be provided at a point equidistant from each lateral edge. Alternatively it may be offset. The method may comprise providing the join at a point equidistant from each lateral edge. Alternatively it may comprise proving the join in an offset lateral position. For example it may be arranged anywhere between 25% and 75% of the width of the pouch. The distance between the left and right lateral edges of the peripheral seal may be the width of the pouch. The distal end of the catheter may be provided at a distance from one left edge that is no more than 20%, 10%, 5% or 1% of the width of the pouch. Thus the join and distal end are provided in locations that make the catheter easy to withdraw and lubricate without spilling lubricant. The provision of a join that is equidistant between the edges ensures the channel volume is restricted and that the fluid level within it is optimised for lubricating the catheter.

The distal end of the catheter may be arranged closer to one (e.g. a left) lateral edge of the peripheral seal than another (e.g. a right) lateral edge of the peripheral seal. In embodiments comprising an interaction region, actuation of the interaction region may preferentially first create an opening in a first (e.g. left) lateral edge of the peripheral seal. In such embodiments, the distal end of the catheter may be arranged closer to the first (e.g. left) lateral edge. In embodiments comprising a tear-away region and a tear start, the location of the tear start may correspond to a lateral edge of the peripheral seal. In such embodiments, the distal end of the catheter may be arranged closer to the lateral edge of the peripheral seal corresponding to the location of the tear start. One or more handling sleeves and/or handling sleeve elements may be arranged at the distal end of the catheter. Thus, the distal end, and corresponding handling sleeves/handling sleeve elements are located next to the first part of the pouch to be opened and is easier to withdraw.

The join may be provided closer to the base of the peripheral seal than an upper edge of the peripheral seal. The distance between the base and the upper edge of the peripheral seal may define the height of the pouch. The distance between the join and base may be at least: 10%; 15%; 20%; 25%; 30%; 35%; 40% or 45% of the height of the pouch. The distance between the join and base may be less than: 50%; 45%; 40%; 35%; 30%; 25%; 20%; or 15% of the height of the pouch. In particular, the join can be arranged at between 20% and 30% of the height of the pouch. The join may be circular. The join may have a radius equivalent less than 15%; 10%; 5%; or 1% the height of the pouch. In a preferred embodiment, the join has a radius of 5% the height of the pouch. Thus, the join can be arranged to ensure the catheter is adequately lubricated and that it defines a channel of suitable size to allow easy withdrawal of the catheter without excessive bending of the catheter or excessive force to be applied.

In one embodiment, the join is provided at a point equidistant from each lateral edge of the pouch and at a distance from the base that is 20-30% of the height of the pouch.

The distal end of the catheter may be provided with a funnel arranged so as not to pass through the channel during withdrawal of the catheter from the pouch. The one or more handling sleeves may also be arranged so as not to pass through the channel during withdrawal of the catheter. The one or more handling sleeves may be no more than: 25%; 20%; 15%; 10%; or 5% of the length of the catheter, but could be longer. Embodiments of the present invention provide a funnel and at least one handling sleeve to allow easy handling of the catheter. These elements, or at least part of them, do not pass through the channel and so are not coated in fluid during withdrawal. This makes the catheter more convenient to use as the fluid is only applied to the necessary parts of the catheter. It also facilitates using less fluid as it is applied in a targeted manner to the catheter.

The angle subtended by the ends of the catheter when curved in the pouch may be at least: 180; 210; 240; 270; 300; 330; or 360 degrees; it may even be at least 420; 480 or 540 degrees. It may be less than 720; 630 or 540 degrees. The catheter may be arranged within the pouch in a curled or coiled configuration either before use, after use, or both before and after use. Prior to use, the catheter may be arranged such that the join is within an area defined by the curl or coil of the catheter. After use, the catheter may be arranged such that the join is not be provided within an area defined by the curl or coil of the catheter. The peripheral seal may confine the catheter in its curved configuration. As such, the catheter is curved, curved or coiled to reduce the size of the pouch and ensure that long catheters can be conveniently and discreetly stored ready for use.

The catheter may be arranged such that during withdrawal at least: 30%; 40%; 50%; 60%; 70% or 80% of the length of the catheter passes through the channel. In one embodiment, 70-80% of the length of the catheter passes through the channel during withdrawal. Advantageously, only the part of the catheter intended to enter the body is lubricated and therefore fluid is not wasted and the catheter is easier to use.

The walls may be formed of any suitable material, such as film materials. The walls may be impermeable to fluids. The walls may be made of a plastics material. The walls may be made from any one or more of: polypropylene (PP), polyethylene terephthalate (PET), low density polyethylene (LDPE), metalized polyester (MET PET)or orientated polypropylene (OPP), for example. The walls may comprise a laminate of two or more layers. Each wall may comprise an inner layer and an outer layer. Each wall may comprise inner and outer layers of polypropylene. Each wall may comprise an inner layer of orientated polypropylene and an outer layer of polypropylene. Each wall may comprise an inner layer of polypropylene and an outer layer of polyethylene, for example. The inner layer and outer layer may have the same thickness. In other embodiments, the inner layer and outer layer may have different thicknesses. The inner layer may have a smaller thickness than the outer layer. In embodiments, the inner layer may have a thickness of between 5-20 microns, or between 10-10 microns, or between 10-15 microns, and the outer layer may have a thickness of between 10-50 microns, or between 20-40 microns, or between 35-45 microns. In one example, the inner layer may have a thickness of approximately 12 microns and the outer layer may have a thickness of approximately 40 microns.

The pouch may be an opaque pouch. The pouch may comprise a transparent or translucent window (preferably corresponding to the position of the fluid reservoir in the pouch and/or a wetting region into which fluid is intended to flow from the fluid reservoir). The catheter and/or one or more handling sleeves may be arranged within the pouch so as to be hidden from view. A fluid reservoir may be provided within the pouch, and the pouch may be configured so as to allow a user to view the fluid reservoir and/or view fluid released from the fluid reservoir.

Thus, the present invention provides for a packaged catheter assembly that is discreet as the contents of the assembly are hidden. The window provides a visual guide so that during use of the catheter, the user can easily identify the fluid reservoir, and/or easily identify that fluid has been successfully released from the reservoir into a wetting region of the pouch to lubricate the catheter prior to use. Whilst the provision of a transparent/translucent window is the preferred approach for allowing a user to view whether fluid has been successfully released from the reservoir, alternative approaches are considered feasible. For example, rather than providing a transparent/translucent window in an opaque wall of the pouch, it is conceived that the functionality could be achieved by providing a transparent/translucent pouch wall, but including an intervening opaque wall, within the pouch provided between the transparent/translucent pouch wall and the catheter (and optionally the reservoir). The intervening wall could include a window, or fluid released from the reservoir could flow between the outer transparent/translucent wall and the intervening wall, providing an indication of successful release.

The pouch may comprise two opaque walls. The window may be provided in one of the opaque walls. The window may be spaced from at least one edge of the pouch or the wall it is provided in. The window may be spaced from all edges of the pouch. The window may be less than: 25%; 20%; 15%; 10% or 5% the total area of the pouch and/or the wall it is provided in. The window may correspond to the size and/or shape of the fluid reservoir. The window may be a smaller size than the fluid reservoir. In one embodiment, the window is the same shape but a smaller size than the fluid reservoir. Thus, in embodiments of the invention, the window advantageously corresponds to the size, shape and position of the fluid reservoir and is provided in a discrete manner, separated from the edges of the pouch and only provided in a section of the pouch's wall.

The window may correspond to the position of an indicator region of the pouch. the indicator region may correspond to a wetting region, which may extend from the base of the pouch to a level at which sufficient fluid has escaped from the pouch to wet the catheter. The indicator region may be configured to indicate successful release of fluid from the reservoir. The indicator region may comprise a fill-level marker indicating sufficient fluid is in the wetting region. Thus the window also provides a visual guide to ensure fluid released from the reservoir has been delivered to a specific area of the pouch (e.g. a channel) or that it has successfully lubricated the catheter.

The pouch may be substantially rectangular in shape with two lateral edges, a base, and an upper edge. The height of the pouch may be defined as the distance between the base and upper edge. The width of the pouch may be defined as the distance between the lateral edges of the pouch. The window may span at least: 20%, 30%; 40%; 50%; 60%; 70%; 80% or 90% the height of the pouch. In one embodiment, the window spans at least 30-40% of the height of the pouch and the window is between 15-20% of the width of the pouch. Thus, the fluid reservoir is easily visible as the window is aligned with it, but simultaneously, the area of the window is minimised to ensure the contents of the pouch remain generally not visible, on account of the fact that but for the window, the pouch is opaque.

The catheter in the pouch is preferably hidden. The catheter may be hidden by the opaque walls of the pouch. The catheter may be hidden by the fluid reservoir. The one or more handling sleeves in the pouch is preferably hidden. The one or more handling sleeves may be hidden by the opaque walls of the pouch. The one or more handling sleeves may be hidden by the fluid reservoir. This is particularly advantageous from the point of view of discretion -the catheter and handling sleeve(s) are likely to appear to observers as some kind of medical device, even if they are not aware of its precise nature; hiding it, either by ensuring that it is arranged out of register with the window, or by hiding it behind another object, for example the fluid reservoir (but optionally a dedicated sheet material, for example) obscures this.

Notably, the fluid reservoir (or other obscuring object) may be opaque, and may have a colour that corresponds to, or contrasts with that of the walls of the pouch. For example, the reservoir (or other obscuring object) may be in the same colour, but a different shade to that of the pouch, or where the opaque pouch has multiple colours, may be a shade of one of those colours. A grey or silver is considered particularly preferable for the reservoir (or other obscuring object) from the point of view of discretion.

One or more handling sleeve, and/or one or more handling sleeve elements, may be opaque and have a colour that corresponds to, or contrasts with, that of the walls of the pouch and/or the catheter. Thus, the handling sleeve can be constructed to be similar to the colour of the pouch and/or catheter and thereby provide a more discrete catheter.

The window may be patterned; i.e. at least part of the window may be provided with a pattern thereon, such as a pattern formed by opaque regions, such as spots, stripes or the like. The pattern may provide a graduated appearance to the window. The pattern may provide a graduated appearance to one or more of the edges the window. The pattern may blend the window into the surrounding packaging of the pouch. Thus, the window is less obvious to the untrained eye and is therefore the packaged catheter assembly (both before and after use) is more discreet for the user to carry with them.

A retaining seal may be provided between the two walls to restrict the movement of the fluid reservoir (which may of course be a sachet as defined above) in the pouch. The retaining seal may be provided outside the curvature of the catheter. The retaining seal may be closer to an upper edge of a peripheral seal of the pouch than a base of the pouch. The retaining seal may extend from an upper edge of a peripheral seal of the pouch.

According to a second aspect of the present invention a method is provided as described in claim 15.

The method may comprise first opening a packaged catheter assembly to provide the catheter and the pouch. The packaged catheter assembly is a packaged catheter assembly according to claim 1, optionally including any of the optional features set out above. Indeed, it may comprise any of the optional features without necessarily including all the features set out in the first aspect above.

The method comprises separating at least one handling sleeve into at least two handling sleeve elements. Thus, the at least one handling sleeve may be separated to provide handling sleeve elements that enable convenient and easy packaging of the catheter.

The method may comprise moving one or more handling sleeve elements along the length of the catheter. Thus, the handling sleeve elements may be easily arranged to assist handling different parts of the catheter.

The catheter may comprise a proximal end for insertion into the body and a distal end. The method may comprise arranging one handling sleeve element, a distal handling sleeve element, at or near to the distal end of the catheter. The method may comprise arranging one handling sleeve element, a proximal handling sleeve element, at or near to the proximal end of the catheter. Thus, the ends of the catheter can be conveniently packaged without touching the used catheter surface itself.

The method may comprise coiling or looping the catheter. The method may comprise receiving at least first and second sections of the catheter within at least one handling sleeve elements, preferably at one handling sleeve element. The method may comprise looping the catheter between the first and second sections of the catheter. The first section may be an end of the catheter, preferably the proximal end. The second section may not be an end of the catheter. The method may comprise arranging a first handling sleeve element along the catheter at a point corresponding to the second section. The method may comprise inserting an end of the catheter into the first handling sleeve element. A second handling sleeve element may be used to insert an end of the catheter into the first handling sleeve element. Preferably, the first handling sleeve element is the distal handling sleeve element. Thus, the catheter can be looped tightly to restrict its size/shape using two or more handling sleeve elements.

The method may comprise forming a knot in the catheter. The method may comprise forming a loop in the catheter using the two or more handling sleeve elements. The method may comprise passing an end of the catheter through the loop so as to form a knot. Preferably, the distal end may be passed through the loop. Thus, the size/shape of the catheter may be further restricted by knotting the looped catheter. **In** addition, both ends of the catheter are retained in position ensuring the catheter is easily repackaged in the pouch.

The method may comprise arranging the distal end next to, or in contact with, the proximal end. The method may comprise fixing the distal end to the proximal end. The method may comprise arranging the catheter in a circular or looping shape. The catheter may comprise a funnel arranged at its distal end. The method may comprise inserting the proximal end into the distal end, or vice versa. The method may comprise inserting the proximal end into the funnel. The method may comprise compressing the funnel to retain the proximal end within it. Thus, the catheter's ends are connected together to form a circular shape which makes the catheter easier to handle and package.

The method may comprise restricting the shape of the catheter using two or more handling sleeve elements. The method may comprise gripping two or more of the two or more handling sleeve elements with one hand. The method may comprise inserting the catheter into the pouch using the two or more gripped handling sleeve elements. Thus, the catheter can be held in a restricted shape using just one hand allowing the other hand to hold the pouch open so as to receive the catheter.

According to a third aspect (not claimed) there is provided a method of using a catheter, wherein the catheter comprises at least one handling sleeve configured to provide at least two handling sleeve elements, the method comprising: introducing the catheter to the body; withdrawing the catheter from the body using at least one handling sleeve of the catheter; and arranging the catheter within a pouch using the at least two handling sleeve elements.

The method may comprise providing a packaged catheter assembly comprising the catheter retained within the pouch. The method may comprise opening the pouch and withdrawing the catheter from the pouch.

Where the catheter is a male urinary catheter, the method may further comprise introducing the catheter by its proximal end into the urethra. The method may comprise releasing urine through a funnel at the distal end of the catheter. The method may further comprise removing the catheter.

In the method of using the catheter, the packaged catheter assembly may be a packaged catheter assembly as defined in general terms, or according the first aspect set out above, optionally including any of the optional features set out above. Indeed, it may comprise any of the optional features without necessarily including all the features set out in the first aspect above.

The method of using the catheter may also optionally include any one of the features as set out in the method of packaging a catheter according to the second aspect above. It may comprise any of the optional features without necessarily including all the features set out in the second aspect above.

### Detailed Description of the Invention

In order that the invention may be more clearly understood one or more embodiments thereof will now be described, by way of example only, with reference to the accompanying drawings, of which:
- Figure 1: is an exploded view of a first embodiment of a packaged catheter assembly;
- Figure 2: is a front view of the sealed assembly of Figure 1;
- Figure 3: is a front view of the packaged catheter assembly of Figures 1 and 2 where fluid has been released into the pouch;
- Figure 4: is a front view of the packaged catheter assembly of Figures 1-3 where the package has been opened;
- Figure 5: is a front view of the packaged catheter assembly of Figures 1-4 where the catheter has been part withdrawn from the pouch;
- Figure 6: is a front view of the packaged catheter assembly of Figures 1-5 where the catheter has been almost fully withdrawn from the pouch;
- Figure 7: is a handling sleeve configured to be separable into two handling sleeve elements;
- Figure 8: is the handling sleeve of Figure 7 separated into two handling sleeve elements
- Figure 9: is the catheter of the packaged catheter assembly of Figures 1-6 where the handling sleeve has been separated into two handling sleeve elements;
- Figure 10: is the catheter of Figure 9 coiled using the two handling sleeve elements prior to re-packaging;
- Figure 11: is a front view of a used and re-packaged catheter assembly of Figures 1-6;

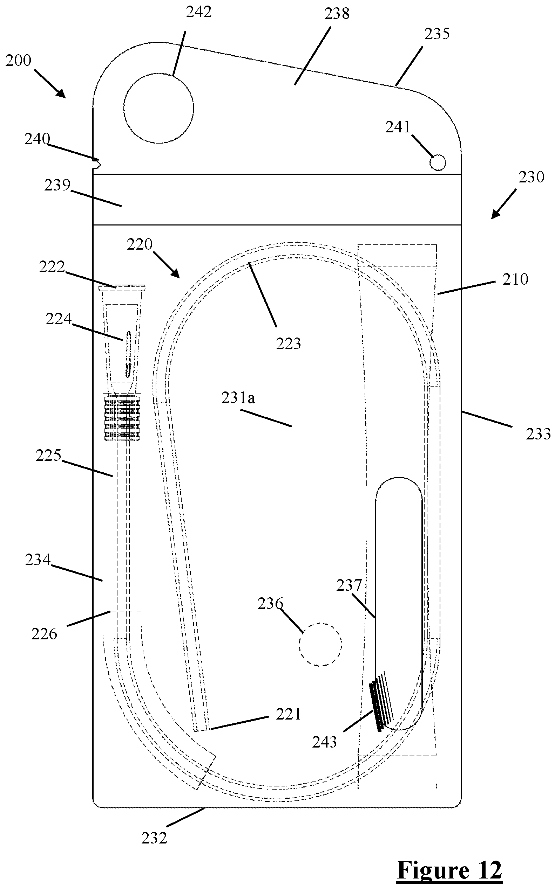

- Figure 12: is a front view of a second embodiment of a packaged catheter assembly; and
- Figure 13: is a schematic magnified view of a part of the inner surface of the handling sleeve of Figure 7.

In the figures, as is conventional, broken lines show hidden features.

The present disclosure concerns a catheter and in particular a handling sleeve for the catheter. It is described in the context of a packaged catheter assembly. The packaged catheter assembly comprises a pouch and the catheter. The catheter is arranged within the pouch. The catheter comprises at least one handling sleeve configured to provide at least two handling sleeve elements after withdrawal of the catheter from the pouch. The two handling sleeve elements can thus be used separately to better manipulate the catheter without touching its surface. In the described embodiment the two handling sleeve elements are initially provided as two halves of a single handling sleeve, the handling sleeve being frangible, to separate into the two handling sleeve elements. Of course it will be immediately understood that in another embodiment the catheter is provided initially with two separate handling sleeves each providing an independent handling sleeve element.

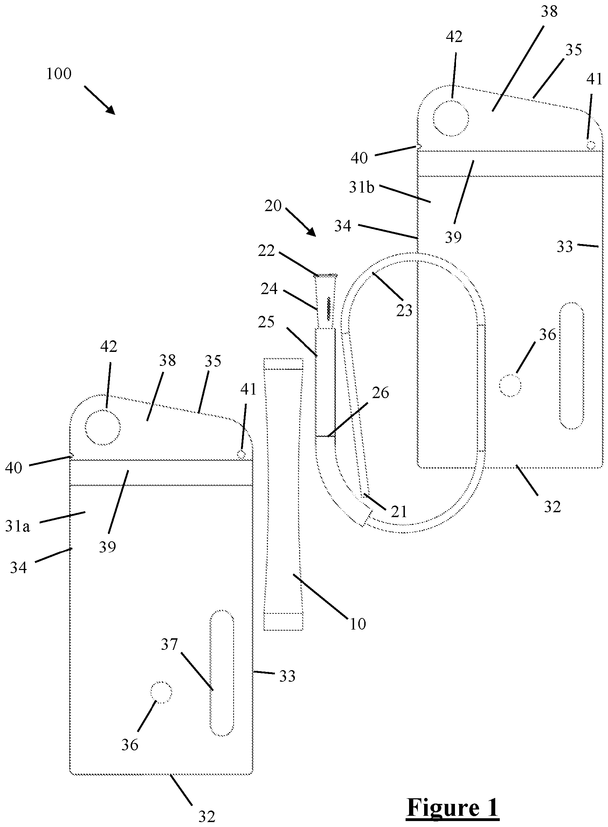

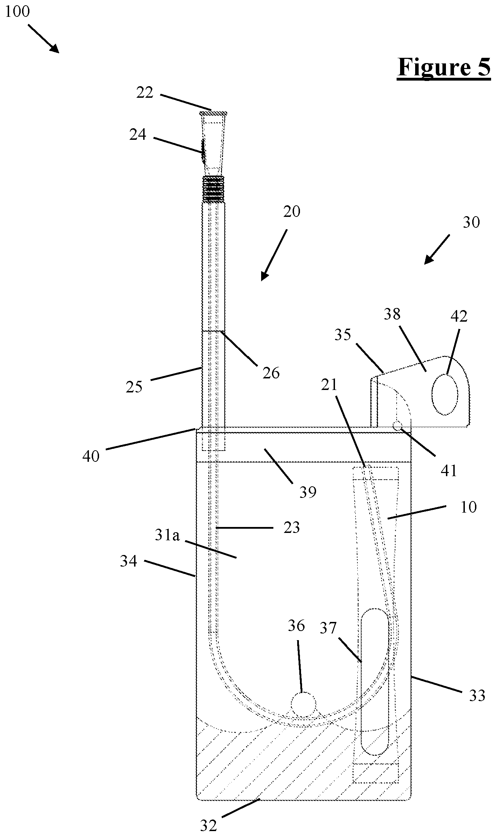

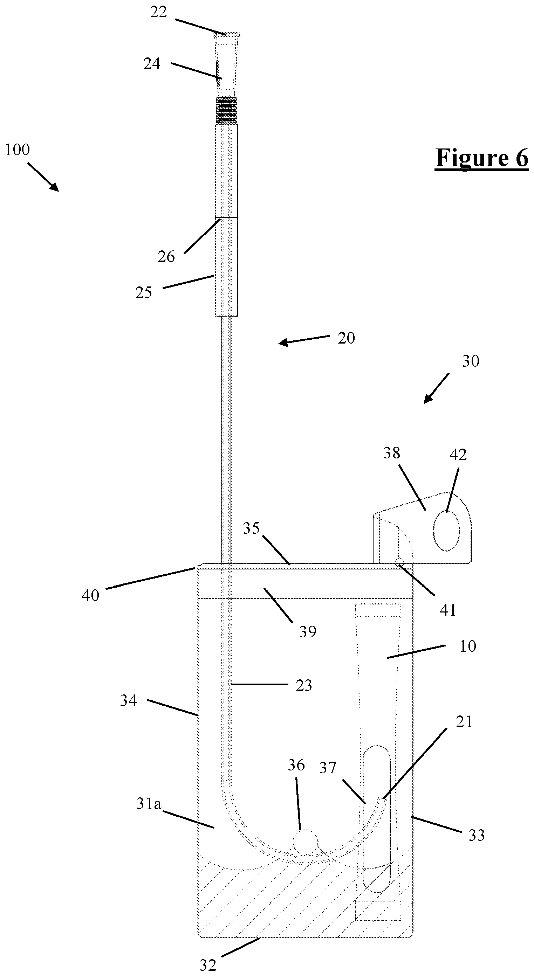

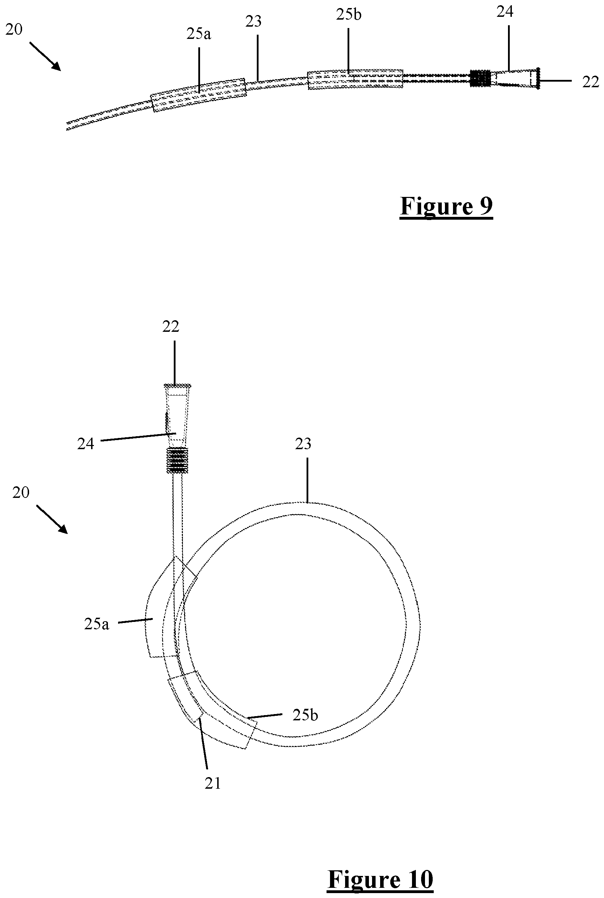

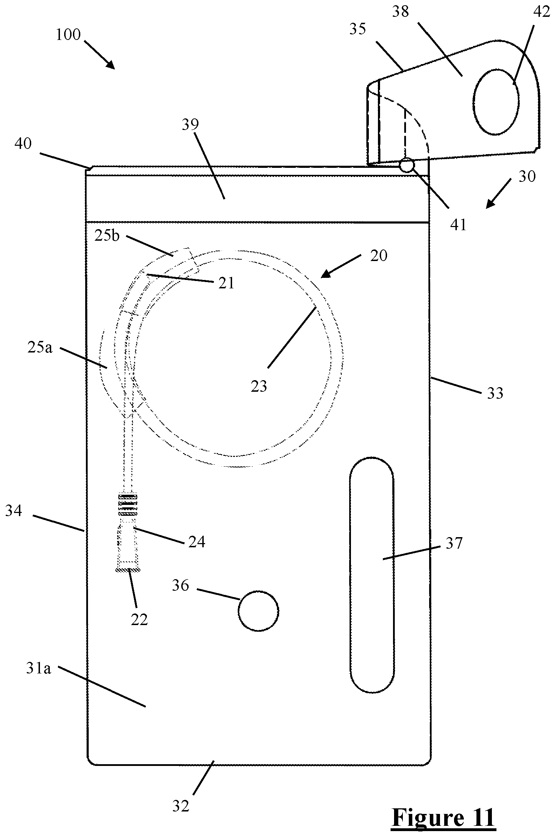

Referring to Figures 1-11, a first embodiment of a packaged catheter assembly 100 is shown.

The assembly 100 comprises a fluid reservoir 10, a male intermittent single-use urinary catheter 20, and a pouch 30. The catheter 20 comprises a proximal end 21 for insertion into the body, a distal end 22, and a flexible tube 23 connecting the two ends 21, 22. The distal end 22 comprises a funnel 24 to guide urine from the bladder out of the tube 23 in use. A handling sleeve 25 is also provided around the tube 23, and it is provided adjacent to the distal end 22. The handling sleeve 25 comprises a point of weakness, which in this embodiment is a line of perforations 26 that extends around a circumference of the handling sleeve 25. The perforations 26 are breakable and as such the handling sleeve 25 is separable into two handling sleeve elements, a proximal element 25a and a distal element 25b, as described below.

In this embodiment, the catheter tube 23 is a hydrophilic thermoplastic elastomer (TPE), and the handling sleeve 25 and handling sleeve elements 25a, 25b are formed from a film of low-density polyethylene (LDPE). The funnel 24 is made from polyvinyl chloride (PVC).

In this embodiment, the pouch 30 comprises a front opaque wall 31a, a rear opaque wall 31b opaque wall, and a peripheral seal joining the periphery of the walls 31a, 31b together to form the pouch. The peripheral seal comprises a base 32, a right lateral edge 33, a left lateral edge 34, and an upper edge 35. The left lateral edge 34 and right lateral edge 33 being defined as the left and right sides of the pouch when viewing the pouch 30 with the rear wall 31b behind the front wall 31a, the base 32 at the bottom of the pouch 30 and the upper edge 35 at the top of the pouch 30. The peripheral seal thus defines a pouch 30 that is generally rectangular and suitably has a width between the lateral edges of between 60-110 mm, for example about 100mm, e.g. 95 mm, and a height from the base 32 to the upper edge 35 of between 100 to 250 mm, for example about 200 mm, e.g. 197 mm. The base 32 defines the bottom of the pouch in use, and the upper edge 35 the top. However, in other embodiments alternative shapes and sizes could be conceived, such as an elliptical or circular pouch.

The pouch also comprises a join 36 between the two walls 31a, 31b. The join 36 is a circular weld provided at point equidistant between the lateral edges 33, 34 of the peripherals seal and at a distance above the base 32 extending from 45 to 55mm. The join 36 may have a radius between 1% and 10% the height of the pouch, for example 5%. In other embodiments the join may be provided in different shapes and sizes, at different locations, or could potentially even be integrated into a continuous seal with the peripheral seal.

The pouch also comprises a window 37 in one of the walls, which allows a user to see inside the pouch. In this particular embodiment, the window 37 is transparent elongate and generally rectangular with a length between 20-50% the height of the pouch 30, for example 33%, and width between 5-25% the width of the pouch 30, for example 12.5%. In this example, the window 37 has semi-circular ends, but in other embodiments they may be any suitable shape, such as flat ends or irregularly shaped ends. The window 37 is arranged parallel to the right lateral edge 33, and a gap is provided between the edge of the window 37 and right lateral edge 33 equivalent to between 5- 20% the width of the pouch, for example 10%. The bottom of the window 37 is separated from the base of the peripheral seal 32 by a distance equal to between 5-20% the height of the pouch, for example 10%. In other embodiments, translucent windows may be used and additionally alternative shapes, sizes and positions or angles of the window are contemplated (for example say at an angle of up to 20 degrees with respect to the edges.

In this particular embodiment, each wall 31a, 31b comprises a plurality of layers of foil; in one specific embodiment a 97 micron 2-layer foil of 12 micron PET and 60 micron LLDPE. In other embodiments, the walls 31a, 31b may only comprise a single layer, and different plastics materials may be used interchangeably.

As mentioned above, the walls 31a, 31b are opaque. This may be achieved by forming each wall 31a, 31b from one or more opaque layers of material or printing an opaque pattern onto one of the layers. The window 37 in the front wall 31a, may be formed by laminating an opaque layer of material onto a transparent wall, wherein the opaque layer has an aperture corresponding to the window 37. Alternatively, a transparent wall may be printed on to block light from passing through the wall, rendering it opaque in all places except for the location of the window 37. Further alternatives may also be contemplated and achieved, for example, providing an aperture corresponding to the window 37 in an opaque wall, and bonding a transparent layer so as to cover the aperture and achieve a continuous front wall 31a with a window 37, or starting with a multi-layer foil with an opaque layer and a transparent layer and removing part of the opaque layer of foil to form the transparent window.

In this particular embodiment, the pouch also comprises an interaction region. The interaction region forms the top part of the pouch 30 and spans between the right lateral edge 33 and left lateral edge 34. The interaction region is used to provide access to the pouch through/near to the upper edge 35. In this embodiment, the interaction region comprises a tapered tear away region 38 and a sealing arrangement 39. The tear-away region 38 comprises a tear start 40 on the left lateral edge, about 2cm below at the point the upper edge 35 and left lateral edge 34 meet, and a tear stop 41 at a point in the pouch 30 about 1cm below where the upper 35 and right lateral 33 edges meet. The distance from the tear stop 41 and the right lateral edge 33 being between 1-10% the width of the pouch, for example 5%. The tear start 40 comprises a notch to provide an area of weakness in the walls 31a, 31b allowing the tear away region 38 to be separated from the pouch 30 by tearing the walls apart from the tear start 40 to the tear stop 41. Thus the tear is provided beneath the location of the upper edge 35 and thus the pouch 30 is opened provided access to its contents. The tear stop 41 comprises a small aperture in the walls 31a, 31b to prevent further tearing of the pouch 30 and separation of the tear away region 38 from the pouch 30. In order to provide the tapered shape, the tear away region 38 extends upwards on the left side approximately 50% more than it does on the right side. The left side of the tear away region 38 comprises a circular aperture 42 sized to allow a finger to pass through and grip the tear away region 38. To ensure the pouch is fully sealed, the front wall 31a and rear wall 31b are sealed to one another around the periphery of the tear stop 41 and circular aperture 42. In other embodiments, other seals such as a zip-lock arrangement may be used to provide access to the pouch, and of course, the sizes and shapes set out above are exemplary.

In this embodiment, the sealing arrangement 39 extends between the left lateral edge 34 and right lateral edge 33 of the peripheral seal just below the tear away region 38. The sealing arrangement 39 comprises first and second sealing members (not shown) provided on an interior surface of the walls 31a, 31b of the pouch 30. In this embodiment, the sealing members comprise corresponding sealing grooves and ridges to allow the sealing members to be press-fit together to form a water-tight seal and pulled apart again if necessary. In other embodiments, a single adhesive sealing member may be used, or a sealing member may be provided on the outside of the pouch to allow the pouch to be folded or rolled and sealed accordingly and of course, alternative sealing mechanisms like a hook-and-loop or hook-and-hook seal or zip-lock could be used.

In this embodiment, the fluid reservoir 10 is an elongate rectangular burstable sachet of water, but in other embodiments any suitable reservoir may be used. The fluid reservoir 10 has a height equal to between 60-90% the height of the pouch 30, for example 80%, and a width between 5-25% the width of the pouch 30, for example 15%. In this embodiment, the fluid contained within the reservoir 10 is water. In other embodiments, many other types of reservoir would be suitable including different shapes and sizes of reservoir, or non-burstable reservoirs and other external sources of fluid. In addition, other fluids could be used, for example lubricants, gels, oils or the like.

In this embodiment, the catheter 20 has a length of between 30-50 cm, for example 40 cm, and the handling sleeve 25 is between 10 and 30% the length of the catheter 20, for example 20-25%, e.g. 11 cm. In this embodiment, the perforations 26 are located at a mid-point along the length of the handling sleeve 25. The distal element 25b therefore constitutes the half of handling sleeve 25 closest to the distal end 22 of the catheter 20, and the proximal element 25a the half of the handling sleeve 25 closest to the proximal end 21 of the catheter 20.

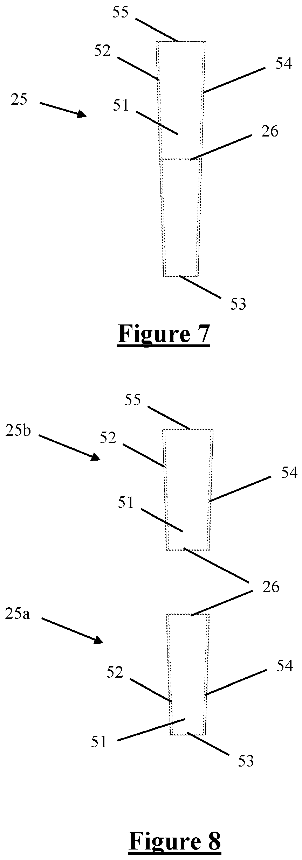

Referring to Figure 7, in this embodiment the handling sleeve 25 is formed from two elongate strips of material (for example, a first strip 51, and a second strip (not shown)) joined along their respective left and right elongate edges, for example by welding, to form the left edge 52 and right edge 54 of the handling sleeve 25. The handling sleeve 25 is a truncated cone with a width at an open proximal end 53 corresponding to the proximal element 25a of 17 mm, and a width at the opposite open distal end 55 corresponding to the distal element 25b of 23 mm. Advantageously, this allows the wider distal end 55 of the handling sleeve 25 to overly the funnel 24 if required, while the thinner proximal end 53 of the handling sleeve 25 prevents the handling sleeve from being removed from the catheter 20. The width of the handling sleeve 25 changes linearly along its length, and as such, its width at the perforations 26 is 20 mm. The elongate strips forming the handling sleeve 25 each have a length of 110 mm and are therefore isosceles trapeziums with a base of 23 mm, a top of 17 mm, and a height of 110 mm. Accordingly, the strips are joined along their legs to form the handling sleeve 25.

In this embodiment, the handling sleeve 25 is perforated across its width, half way along its length, to form the perforations 26 prior to joining the elongate edges 52, 54, so as to provide the two separable handling sleeve elements 25a, 25b. In this embodiment, the perforations 26 comprise a row of elongate holes extending around the circumference of the handling sleeve 25. The holes have a width and length perpendicular to the width and length of the handling sleeve 25 respectively. In this embodiment, the holes are slits and as such they are one dimensional and have an aspect ratio (length:width) that is much greater than 2:1 and effectively 1:0.

In this embodiment, the holes are separated by a distance that is no more than the length of each hole, and in this example the holes are separated by 1 mm. In this embodiment, the length of each hole is 2 mm. As such, the perforations 26 provide for a point of weakness in the handling sleeve 25 that is sufficient to allow easy separation of the handling sleeve 25 into two handling sleeve elements 25a, 25b. Furthermore, as they are slits, they reduce the likelihood of inadvertent contact with the catheter 20 through the holes when handling the catheter 20. This helps to ensure the catheter 20 remains clean and sterile prior to use.

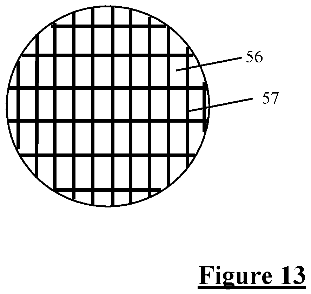

In this embodiment, the catheter 20 is provided with a hydrophilic coating, this is activated when it contacts the fluid held in the reservoir 10. In other embodiments, any suitable catheter length or type may be used. Referring to Figure 13, in this embodiment the inner surface 56 of the handling sleeve 25 is hydrophobic and comprises an embossed pattern 57. This reduces friction between the catheter 20 and the handling sleeve 25 allowing easier arrangement of the sleeve along the catheter 20 during use. In this example, the embossed pattern 57 is a linen pattern.

The catheter 20 is arranged within the pouch 30 in a curved and coiled configuration, with the distal end 22 adjacent to the inner left lateral edge 33 near the upper 35 edge of the pouch 30, just beneath the sealing arrangement 39. The catheter 20 extends down the inside of the pouch 30 into a broadly elliptical anti-clockwise coil (as viewed with left lateral edge 34 on the left, and upper edge 35 at the top). The coil having a single turn around the inner perimeter of the left lateral edge 34, base 23, and right lateral edge 33 of the pouch, with the proximal end 21 of the catheter 20 resting on the inside of the catheter's coil, adjacent to the left lateral edge 34 and base 32 of the pouch. Notably, the majority of the handling sleeve 25 is arranged above the join 36, and as such, the perforations 26 are arranged above the join 36. As the catheter 20 curves and extends from left to right, the catheter tube 23 runs underneath the join, in a channel defined by the join and the internal base of the peripheral seal, then runs upwards to the side of the join, before returning over the top of the join and finally extending downwardly such that the proximal end 21 is lower than and on the left side of the join 36. In short, the catheter 20 is coiled (or curled) around the join 36 (albeit not tightly coiled). In other embodiments, the position of the catheter 20 may be different, in particular, the catheter 20 may not coil or curl but may simply curve within the pouch 30. On the other hand, embodiments of the invention may have a catheter 20 that has multiple turns of coil.

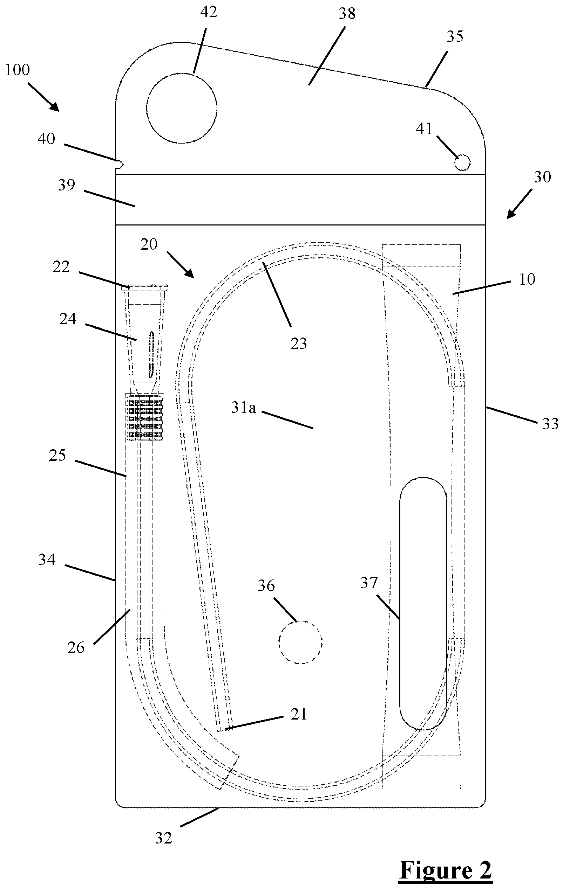

Referring to Figure 2, the pouch 30 is formed by first providing the two walls 31a, 31b which are to define the pouch 30 itself, and the front wall 31a comprising the transparent window 37 as described above and produced by one of the techniques described above. In this embodiment, the walls 31a, 31b are sized to correspond to the final shape of the pouch 30, however in other embodiments, the walls may extend outside the pouch 30 as required.

In this embodiment, the base 32, right lateral edge 33 and left lateral edge 34 are the first to be formed by welding the two walls 31a, 31b together, leaving the pouch 30 with an open upper edge 35. The catheter 20 is then arranged within the pouch 30 in a curved and coiled configuration, as set out above

In this embodiment, the fluid reservoir 10 is also placed within the pouch 30 at a position corresponding to the window 37. The reservoir 10 is placed over the catheter 20 so that the reservoir 10 is visible through the window 37 and the reservoir 10 obstructs any view of the catheter 20 through the window 37. In other embodiments, the reservoir 10 may not need to be placed over the catheter 20 as the position of the catheter 20 in the pouch 30 may not correspond to the window 37.

In this embodiment, the first and second sealing elements (not shown) are then attached to the inside of the walls 31a, 31b just above the coiled catheter 20, and the peripheral seal is completed by forming its upper edge 35 by welding the walls 31a, 31b together along the upper edge 35. This seals the catheter 20 and reservoir 10 within the pouch 30. In other embodiments, the sealing elements may be provided to the walls prior to any other assembly of the pouch 30, or sealing of the peripheral seal.

In this embodiment, the join 36 is then formed by welding the walls 31a, 31b together. Because the catheter 20 is coiled around the inner perimeter of the pouch 30, the join 36 is located within the coil of the catheter 20. Therefore, part of the catheter tube 23 passes between the join 36 and the base 32 of the pouch. In other embodiments, the join 36 may be provided before the peripheral seal, or at any point of the assembly process.

Finally, in this embodiment, the tear-start 40, tear-stop 41, and aperture 42 may be formed by cutting out the appropriate sections of the pouch 30. Use of a hot punch can simultaneously bond the walls 31a, 31b around the periphery of the cut-out regions to ensure the pouch 30 remains fully sealed.

As will be appreciated, the above is an example and in other embodiments the pouch 30 may be formed in different ways with the steps performed in different orders. For example, the bottom could be open and the catheter 20 and water reservoir 10 introduced from the bottom before the base seal 32 is provided. Or, for example, in an industrially applied process, two webs of wall material may have one half of the sealing arrangement applied to (an interior surface of) each of them, the catheter 20 and reservoir 10 may be arranged between opposing interior surfaces of the webs of wall material, then potentially in a single action, a hot die may join and form the walls 31a, 31b by forming the peripheral seals 32, 33, 34, 35, simultaneously forming the tear start 40, forming the join 36 and punching out and sealing the aperture 42 and tear stop 41 in the interaction region 38. Furthermore, while the peripheral seal and join 36 have been described as formed by a weld, any suitable means of joining the walls could be used, for example, an adhesive may be used.

Referring to Figures 2-9, this first embodiment of a packaged catheter assembly 100 may be unpackaged as described below.

First, the fluid reservoir 10 is located within the pouch 30 with the assistance of the window 37, though which the reservoir 10 can be seen. Fluid is then released from the reservoir 10 into the pouch 30. In this embodiment, the reservoir 10 is a burstable sachet and fluid may be released from it by applying manual pressure to the reservoir 10. The window 37 provides a visual reference to show when the reservoir has been successfully burst and fluid released into the pouch 30. In particular, it can be seen that sufficient fluid has been released to fill the channel defined by the weld 36 and the internal base 32 of the peripheral seal. In other embodiments, the fluid may be released from other means.

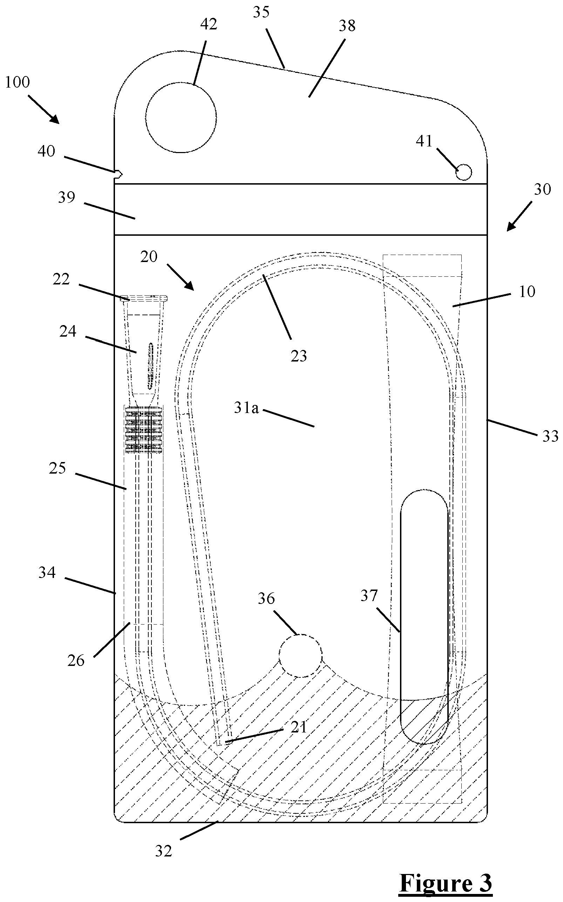

Referring to Figures 3-6, the fluid released from the reservoir 10 collects in a pool at the bottom of the pouch 30, as shown by the hashing. In this embodiment, the fluid fills the pouch 30 up to a fill level corresponding to the join 36. As such, in this embodiment, the join 36 and base 32 define a channel that is completely full of fluid. However, in other embodiments, the fluid may only partially fill the channel, for example filling the channel between 60-95%, or may fill the pouch above the level of the join 36.

The released fluid is also visible through the window 37, as the base of the window 37 corresponds to a location on the pouch 30 that is slightly lower than the join 36. In other embodiments, the window may comprise an indicator region that shows the presence of fluid in the pouch. For example, a fluid-sensitive colour changing strip such that direct visualisation of the fluid is not required. A fill-level marker could also be provided to show that sufficient fluid has been released.

In this embodiment, the contents of the pouch 30 are then accessed by tearing the top of the pouch 30 using the tear away region 38. The aperture 42 is grasped in one hand, and the rest of the pouch 30 in another, and the tear away region 38 is torn from the tear start 40 to the tear stop 41. Thus, the pouch is torn between the left lateral edge 34 and right lateral edge 33 at a location below the upper edge 35, as such, an opening is formed in the pouch 30 that may be used to access its contents. Advantageously, the tear away region 38 is not fully separated from the pouch 30 which reduces the number of separate parts and makes the pouch 30 easier to handle. Other embodiments may feature other ways to access the pouch for example a zip-lock seal.

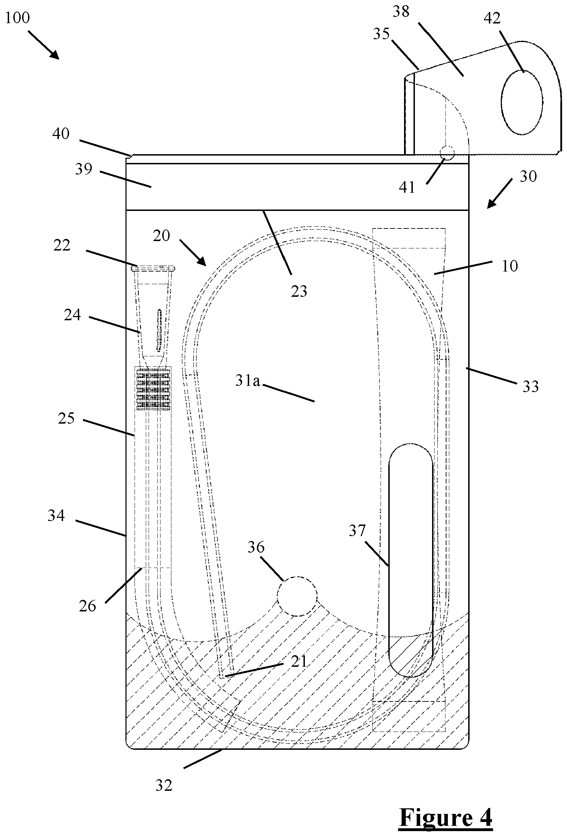

Referring to Figures 4-6, the catheter 20 may then be withdrawn from the pouch 30. As described above, in this embodiment the catheter 20 is coiled in the pouch 30, and the distal end 22 is provided adjacent the upper edge 35 of the pouch 30. As such, the distal end 22 is above the fill line of the fluid released into the pouch 30 and is not wetted by the fluid. The distal end 22 therefore provides a dry and easy to access point which may be used to withdraw the catheter 20 from the pouch 30. In this embodiment, the majority of the handling sleeve 25, i.e. between 60-100%, for example 70% of the sleeve 25 is also above the fill line and is not wetted. Thus, the sleeve 25 also provides a convenient dry surface to handle the catheter 20. In particular, the distal handling sleeve element 25b is entirely above the fill line and is therefore dry.

In this embodiment, the distal end 22 is pulled upwards through the opening in the upper edge 35 of the pouch 30. As the distal end 22 is pulled out of the pouch 30, the catheter tube 23 is uncoiled and also pulled upwards towards the upper edge 35 of the pouch 30. However, as the join 36 is arranged within the coil of the catheter 20, the tube 23 is prevented from moving directly upwards by the join 36. The join 36 therefore causes the tube 23 to be withdrawn from the pouch 30 along a path that includes passing through the channel defined by the join 36 and base 32. As such, the entire remainder of the tube 23 to the proximal tip 21 passes though the pool of fluid in the channel and the hydrophilic coating of the catheter 20 is activated by the fluid. Furthermore, the proximal end 21 of the catheter 20 is the final part of the catheter 20 to pass through the channel and be withdrawn from the pouch 30. Thus, the catheter 20 is activated by the fluid as it is withdrawn and the withdrawal is convenient and easy as only a dry part of the catheter 20 needs to be touched during withdrawal. As the catheter 20 passes through a pool of fluid it is more completely wetted than would be the case for a fluid held within another medium such as a foam. In other embodiments, the catheter 20 need not be withdrawn directly upwards but may be pulled out to sideways from the pouch, for example from an opening provided at the side of the pouch, but, of course, above the weld, so as to reduce the prospect of spillage. Further the catheter 20 may not directly touch the join 36 and may instead be prevented from moving upwards by the convergence of the walls 31a, 31b as they approach the join 36.

Once the catheter is fully withdrawn, the catheter may be used by inserting the proximal end 21 into the urethra until urine begins to flow through the catheter 20. The (largely dry) handling sleeve 25 assists in the insertion. The urine may then be directed out of the catheter 20 by the funnel 24.

Referring to Figures 7-11, the catheter 20 may be placed back inside the pouch 30 in a coiled configuration after use. The handling sleeve 25 including proximal element 25a and distal element 25b, and funnel 24 provide for dry surfaces that can be used to re-coil the catheter 20 and place it into the pouch 30. The catheter 20 may be repackaged back into the pouch 30 as described below.

Referring to Figures 7-9, the handling sleeve 25 may be separated into the proximal element 25a and distal element 25b. This may be done before or after use of the catheter 20 as required by the user. In this embodiment, to separate the handling sleeve 25, the perforations 26 may be broken about the circumference of the handling sleeve 25. The proximal element 25a and distal element 25b are thereby provided on the catheter 20. In this embodiment, each handling sleeve element, the proximal element 25a and distal element 25b, may be independently moved along the length of the catheter 20 to aid ease of use.

In this embodiment, the catheter 20 is then looped and knotted to restrict its size/shape and allow for easy repackaging. Firstly, the proximal element 25a is arranged at a first section of the catheter close to, or at, the proximal end of the catheter 20, and the distal element 25b is arranged at a second section of the catheter 20 between a midpoint and the distal end of the catheter 20. Secondly, the proximal element 25a may be used to create a loop in the catheter 20 between the first and second sections.

The proximal element 25a may then be used to insert the proximal end of the catheter 20 into the distal element 25b. The proximal end of the catheter 20 may thereby be received by, and retained within, the distal element 25b, next to the second section of the catheter 20. Thus, the handing sleeve elements 25a, 25b are used to tightly loop the catheter 20. In other embodiments, multiple loops may be achieved to further restrict the size/shape of the catheter 20 as required.

To further restrict the size/shape of the catheter 20, in this embodiment, the catheter 20 is then knotted. A knot is formed in the catheter 20 by passing the distal end of the catheter 20 through the loop. In this embodiment, this is done by holding the distal element 25b to retain the looped shape of the catheter 20, and then passing the funnel 24 through the loop using the funnel 24 itself (which of course is not inserted into the urethra in use, and is therefore clean and not provided with a hydrophilic surface and therefore not slippery, and as such is easy to grasp). Thus, the size/shape of the catheter may be further restricted by knotting the looped catheter.

Following looping and knotting, the catheter 20 may be held in this form using a single hand grasping the distal element 25b. The pouch 30 may then be held in the user's other hand, and the catheter 20 placed within the pouch 30. Advantageously, the loop and knot prevent the ends of the catheter 20 from getting caught on the sides of the pouch 30 and allow the catheter to easily pass through the opening in the upper edge 35 of the pouch 30.

The sealing arrangement 39 can then be used to reseal the pouch 30 between the left lateral edge 34 and right lateral edge 33 by pressing together the two corresponding sealing elements (not shown). This provides for a more convenient disposal of the catheter 20 after use and ensures the fluid released into the pouch 30 does not subsequently leak out of the pouch 30. In other embodiments, alternative sealing arrangements may be used, such as an external flap with adhesive that allows the top of the pouch 30 to be rolled or folded and sealed. Alternatively, no sealing arrangement may be used where the pouch 30 is intended to be disposed of immediately after use.

Referring to Figure 8, a second embodiment of a packaged catheter assembly 200 is shown. The second embodiment shares many of the features of the first embodiment described above, and so only differences in the features are described below, and like numerals are used to denote like features.

In this embodiment, the join 236 is provided a location offset from the midpoint between the right lateral edge 233 and left lateral edge 234. In particular, the join is provided at a distance from the right lateral edge 234 that is 20-45% the width of the pouch 230, for example, 35%. The join 236 provides a second function as a retaining seal to restrict the movement of the reservoir 210 in the pouch 230, sandwiching the reservoir 210 between the join 236 and the right lateral edge 234. The join 236 therefore ensures the window 237 corresponds to the position of the reservoir 210 regardless of the movement of the pouch 230 as it is carried around by a user, ensuring both that the catheter 223 remains hidden and that the reservoir 210 can be identified easily to release its fluid. In other embodiments, the function of the retaining seal may be provided by a separate seal between the walls 231a, 231b. For example, a separate seal extending downwards from the sealing arrangement 239, or up from the base 232.

In this embodiment, the front wall 231a also comprises a pattern 243 that corresponds to the edge of the window 237. The pattern 243 is only shown on part of the window, but may of course extend over the entirety of it and comprises a graduated appearance that softens the boundary between the transparent window 237 and the rest of the opaque front wall 231a. In this embodiment, the pattern is a series of evenly spaced opaque lines with graduated thicknesses, getting thinner as they approach the centre of the window 237. As such, the pattern 243 makes the window 237 less visually obvious and therefore makes the pouch 230 more discreet. In other embodiments, the pattern 243 could be any suitable pattern, for example opaque circles of graduated size, and may be complemented by or integrate with surface patterns or colour applied to the reservoir 210.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments or that one or more embodiments necessarily include logic for deciding, with or without user input or prompting, whether these features, elements, and/or steps are included or are to be performed in any particular embodiment.

The one or more embodiments are described aboe by way of example only. Many variations are possible without departing from the scope of protection afforded by the appended claims.

## Claims

1. A packaged catheter assembly comprising a pouch (30) and a catheter (20), wherein: the catheter is arranged within the pouch; and the catheter comprises at least one handling sleeve (25) formed of a film material and configured to separate into at least two handling sleeve elements (25a, 25b) after withdrawal of the catheter from the pouch.

2. A catheter assembly as claimed in claim 1 wherein the catheter comprises one handling sleeve configured to provide at least two handling sleeve elements wherein each handling sleeve element corresponds to 30-70% of the length of the handling sleeve.

3. A catheter assembly as claimed in any preceding claim wherein at least one of the at least two handling sleeve elements is configured to receive two or more sections of the catheter simultaneously.

4. A catheter assembly as claimed in any preceding claim the at least one of the at least two handling sleeve elements may be arrangeable to retain the catheter in a looped or coiled configuration.

5. A catheter assembly as claimed in any proceeding claim wherein at least one handling sleeve comprises a point of weakness comprising one or more perforations provided about a circumference of the respective handling sleeve.

6. A catheter assembly as claimed in claim 5 wherein the perforations comprise a row of holes wherein the holes are separated by a distance that is no more than the length of each hole in a direction parallel to the circumference of the respective handling sleeve, and wherein each hole is a slit.

7. A catheter assembly as claimed in claim 5 or claim 6 wherein the catheter comprises one handling sleeve and the handling sleeve is separable at the perforations to provide two handling sleeve elements.

8. A catheter assembly as claimed in any preceding claim wherein the inner surface of the or each handling sleeve comprises an embossed pattern.

9. A catheter assembly as claimed in any preceding claim wherein at least one handling sleeve forms a sheath around at least part of the catheter, and at least one of the at least two handling sleeve element forms a sheath around only part of the catheter and wherein at least one of the at least two handling sleeve elements are independently movable along the length of the catheter.

10. A catheter assembly as claimed in any preceding claim wherein the catheter comprises a proximal end for insertion into the body and a distal end, a funnel at the distal end of the catheter, and at least one handling sleeve is arranged adjacent to the funnel prior to use.

11. A catheter assembly as claimed in any preceding claim wherein the catheter comprises a proximal end for insertion into the body and a distal end, and the at least two handling sleeve elements comprise a proximal element arrangeable at or close to the proximal end and a distal element arrangeable at or close to the distal end, wherein the distal element is further arrangeable between a midpoint and the distal end of the catheter, and wherein the distal element is configured to receive the proximal end of the catheter.

12. A catheter assembly as claimed in any preceding claim wherein at least one handling sleeve has a width measured perpendicular to the axis of the catheter that is constant and wherein the at least one handling sleeve is formed of one or more strips of material joined together along their edges.

13. A catheter assembly as claimed in any of claims 1 to 11 wherein the at least one handling sleeve is conical.

14. A catheter assembly as claimed in any preceding claim wherein the catheter is a male urinary catheter.

15. A method of packaging a catheter, the catheter (20) comprising at least one handling sleeve, the method comprising: providing the packaged catheter assembly of any of claims 1 to 14 in an unpackaged state; separating at least one handling sleeve (25) into at least two handling sleeve elements (25a, 25b); and arranging the catheter within a pouch (30) using the handling sleeve elements.

## Patentansprüche

1. Eine verpackte Katheteranordnung, aufweisend einen Beutel (30) und einen Katheter (20), wobei der Katheter innerhalb des Beutels angeordnet ist; und der Katheter zumindest eine Handhabungshülse (25) aufweist, die aus einem Folienmaterial gebildet und dazu ausgebildet ist, sich nach dem Herausziehen des Katheters aus dem Beutel in zumindest zwei Handhabungshülsenelemente (25a, 25b) zu trennen.

2. Katheteranordnung nach Anspruch 1, wobei der Katheter eine Handhabungshülse aufweist, die dazu ausgebildet ist, zumindest zwei Handhabungshülsenelemente bereitzustellen, wobei jedes Handhabungshülsenelement 30-70 % der Länge der Handhabungshülse entspricht.

3. Katheteranordnung nach einem der vorhergehenden Ansprüche, wobei zumindest eines der zumindest zwei Handhabungshülsenelemente dazu ausgebildet ist, zwei oder mehr Abschnitte des Katheters gleichzeitig aufzunehmen.

4. Katheteranordnung nach einem der vorhergehenden Ansprüche, wobei zumindest eines der zumindest zwei Handhabungshülsenelemente so angeordnet werden kann, dass der Katheter in einer geschlungenen oder gewundenen Ausbildung zurückgehalten wird.

5. Katheteranordnung nach einem der vorstehenden Ansprüche, wobei zumindest eine Handhabungshülse eine Schwachstelle aufweist, die eine oder mehrere Perforationen umfasst, die um den Umfang der jeweiligen Handhabungshülse herum angeordnet sind.

6. Katheteranordnung nach Anspruch 5, wobei die Perforationen eine Reihe von Löchern aufweisen, wobei die Löcher durch einen Abstand getrennt sind, der nicht größer ist als die Länge jedes Lochs in einer Richtung parallel zu dem Umfang der jeweiligen Handhabungshülse, und wobei jedes Loch ein Schlitz ist.

7. Katheteranordnung nach Anspruch 5 oder 6, wobei der Katheter eine Handhabungshülse aufweist und die Handhabungshülse an den Perforationen trennbar ist, um zwei Handhabungshülsenelemente bereitzustellen.

8. Katheteranordnung nach einem der vorhergehenden Ansprüche, wobei die innere Oberfläche der oder jeder Handhabungshülse ein Prägemuster aufweist.

9. Katheteranordnung nach einem der vorhergehenden Ansprüche, wobei zumindest eine Handhabungshülse eine Hülle um zumindest einen Teil des Katheters bildet und zumindest eines der zumindest zwei Handhabungshülsenelemente eine Hülle um lediglich einen Teil des Katheters bildet und wobei zumindest eines der zumindest zwei Handhabungshülsenelemente unabhängig entlang der Länge des Katheters beweglich ist.

10. Katheteranordnung nach einem der vorhergehenden Ansprüche, wobei der Katheter ein proximales Ende zum Einführen in den Körper und ein distales Ende, einen Trichter an dem distalen Ende des Katheters und zumindest eine Handhabungshülse aufweist, die vor der Verwendung neben dem Trichter angeordnet ist.

11. Katheteranordnung nach einem der vorhergehenden Ansprüche, wobei der Katheter ein proximales Ende zum Einführen in den Körper und ein distales Ende aufweist und die zumindest zwei Handhabungshülsenelemente ein proximales Element, das an oder nahe dem proximalen Ende anordenbar ist, und ein distales Element aufweisen, das an oder nahe dem distalen Ende anordenbar ist, wobei das distale Element ferner zwischen einem Mittelpunkt und dem distalen Ende des Katheters anordenbar ist und wobei das distale Element dazu ausgebildet ist, das proximale Ende des Katheters aufzunehmen.

12. Katheteranordnung nach einem der vorhergehenden Ansprüche, wobei zumindest eine Handhabungshülse eine senkrecht zu der Achse des Katheters gemessene Breite aufweist, die konstant ist, und wobei die zumindest eine Handhabungshülse aus einem oder mehreren Materialstreifen gebildet ist, die entlang ihrer Kanten miteinander verbunden sind.

13. Katheteranordnung nach einem der Ansprüche 1 bis 11, wobei die zumindest eine Handhabungshülse konisch ist.

14. Katheteranordnung nach einem der vorhergehenden Ansprüche, wobei der Katheter ein männlicher Blasenkatheter ist.

15. Verfahren zum Verpacken eines Katheters, wobei der Katheter (20) zumindest eine Handhabungshülse aufweist, wobei das Verfahren aufweist: Bereitstellen der verpackten Katheteranordnung nach einem der Ansprüche 1 bis 14 in einem unverpackten Zustand; Trennen zumindest einer Handhabungshülse (25) in zumindest zwei Handhabungshülsenelemente (25a, 25b); und Anordnen des Katheters in einem Beutel (30) unter Verwendung der Handhabungshülsenelemente.

## Revendications

1. Un ensemble à cathéter emballé comprenant une poche (30) et un cathéter (20), dans lequel : le cathéter est disposé dans la poche ; et le cathéter comprend au moins un manchon (25) de manipulation en une matière de film et configuré pour se séparer en au moins deux éléments (25a, 25b) de manchon de manipulation après retrait du cathéter de la poche.

2. Un ensemble à cathéter tel que revendiqué à la revendication 1, dans lequel le cathéter comprend un seul manchon de manipulation configuré pour donner au moins deux éléments de manchon de manipulation, dans lequel chaque élément de manchon de manipulation représente de 30 à 70 % de la longueur du manchon de manipulation.

3. Un ensemble à cathéter tel que revendiqué dans n'importe quelle revendication précédente, dans lequel au moins l'un des au moins deux éléments de manchon de manipulation est configuré pour recevoir deux ou plusieurs parties du cathéter simultanément.

4. Un ensemble à cathéter tel que revendiqué dans n'importe quelle revendication précédente, le au moins un des au moins deux éléments de manchon de manipulation pouvant être disposé pour retenir le cathéter dans une configuration en boucle ou en bobine.

5. Un ensemble à cathéter tel que revendiqué dans n'importe quelle revendication précédente, dans lequel au moins un manchon de manipulation comprend un point de faiblesse comprenant une ou plusieurs perforations prévues autour d'une circonférence du manchon de manipulation respectif.

6. Un ensemble à cathéter tel que revendiqué à la revendication 5, dans lequel les perforations comprennent une rangée de trous, dans lequel les trous sont séparés d'une distance, qui n'est pas plus grande que la longueur de chaque trou dans une direction parallèle à la circonférence du manchon de manipulation respectif, et dans lequel chaque trou est une fente.

7. Un ensemble à cathéter tel que revendiqué à la revendication 5 ou la revendication 6, dans lequel le cathéter comprend un seul manchon de manipulation et le manchon de manipulation peut être séparé aux perforations pour donner deux éléments de manchon de manipulation.

8. Un ensemble à cathéter tel que revendiqué dans n'importe quelle revendication précédente, dans lequel la surface intérieure du ou de chaque manchon de manipulation comprend un motif embossé.

9. Un ensemble à cathéter tel que revendiqué dans n'importe quelle revendication précédente, dans lequel au moins un manchon de manipulation forme une gaine autour d'au moins une partie du cathéter, et au moins l'un des au moins deux éléments de manchon de manipulation forme une gaine autour de seulement une partie du cathéter et dans lequel au moins un des au moins deux éléments de manchon de manipulation sont mobiles indépendamment suivant la longueur du cathéter.

10. Un ensemble à cathéter tel que revendiqué dans n'importe quelle revendication précédente, dans lequel le cathéter comprend une extrémité proximale pour insertion dans le corps et une extrémité distale, un entonnoir à l'extrémité distale du cathéter, et au moins un manchon de manipulation est disposé en étant contigu de l'entonnoir avant utilisation.

11. Un ensemble à cathéter tel que revendiqué dans n'importe quelle revendication précédente, dans lequel le cathéter comprend une extrémité proximale pour insertion dans le corps et une extrémité distale, et les au moins deux éléments de manchon de manipulation comprennent un élément proximal pouvant être disposé à l'extrémité proximale ou près de l'extrémité proximale et un élément distal pouvant être disposé à l'extrémité distale ou près de l'extrémité distale, dans lequel l'élément distal peut être disposé en outre entre un point médian et l'extrémité distale du cathéter, et dans lequel l'élément distal est configuré pour recevoir l'extrémité proximale du cathéter.

12. Un ensemble à cathéter tel que revendiqué dans n'importe quelle revendication précédente, dans lequel au moins un manchon de manipulation a une largeur, mesurée perpendiculairement à l'axe du cathéter, qui est constante, dans lequel le au moins un manchon de manipulation est formé d'une ou de plusieurs bandes de matière jointes ensemble le long de leurs bords.

13. Un ensemble à cathéter tel que revendiqué dans n'importe quelle revendication 1 à 11, dans lequel le au moins un manchon de manipulation est conique.

14. Un ensemble à cathéter tel que revendiqué dans n'importe quelle revendication précédente, dans lequel le cathéter est un cathéter urinaire masculin.

15. Un procédé d'emballage d'un cathéter, le cathéter (20) comprenant au moins un manchon de manipulation,
le procédé comprenant : se procurer l'ensemble à cathéter emballé de l'une quelconque des revendications 1 à 14 dans un état non emballé ; séparer au moins un manchon (25) de manipulation en au moins deux éléments (25a, 25b) de manchon de manipulation ; et mettre le cathéter dans une poche (30) en utilisant les éléments de manchon de manipulation.
